# EUROPEAN PATENT APPLICATION

(11) **EP 3 827 735 A1**
(43) Date of publication of application: **02.06.2021**
(21) Application number: 19383058.5
(22) Date of filing: 28.11.2019
(51) Int. Cl.: A61B 3/024, A61H 5/00

(54) **DEVICE, METHOD AND COMPUTER PROGRAMS FOR VISUAL FIELD REHABILITATION**

(71) Applicant: Health E Health Coorp, SL, 31192 Navarra (ES)
(72) Inventor: SANTOS OLLOQUI, José, 31192 Mutilva (ES); MURIE FERNÁNDEZ, Manuel, 31192 Mutilva (ES); IZCO ZARATIEGUI, Jesus Maria, 31192 Mutilva (ES)
(74) Representative: Juncosa Miró, Jaime

(57) **Abstract**

A device, method and computer programs for visual field rehabilitation are disclosed. The visual field rehabilitation device comprises a visual field capture component including a case and a fastening element allowing the case to be tied up to the face of a user. An indicator device receives an input from the user when the user identifies a light stimulation on a portable computer device. One lens and a support for the lens are also included. The support is configured to locate the lens facing the eyes of the user via movement thereof. The lens comprises a plane-convex lens with a spherical geometry, whereby the lens provides a complete view of the screen while the user is carrying out visual field rehabilitation exercises. A software application is configured to capture said input performed by the user and to automatically modify the visual rehabilitation exercises, independently for each eye, based on a result of said capture.

## Description

### Technical Field

The present invention is directed, in general, to the field of optical devices. In particular, the invention relates to a visual field rehabilitation device, to a method for visual field rehabilitation and to computer programs thereof. The invention is particularly suitable for patients with acquired brain damage, e.g. people who have had a stroke.

### Background of the Invention

A known device in this field is the document WO2018069555-A1 which discloses a kit for adapting a virtual reality headset and method for using same for simple and economical visual neurorehabilitation, both diagnosis and rehabilitation. The kit comprises a mask with two openings opposite the headset screens, the lenses of the headset are fastened to the mask, as well as two cameras and infra-red light means directed towards the user; the infra-red light and the cameras are connected to a control device that in turn comprises data storage media and a processor, the data storage media including a user eye-tracking program and a campimetry program. The method comprises steps for diagnosing losses in visual field: stimulation, tracking the glance of the user to a certain area, storing the position of the center of the pupil, and obtaining a map of the visual field of the user.

WO2018107108-A1 discloses a method enabling the determination of a subject's visual field perimetry using virtual reality with one or more computing devices, such as a mobile computing device, for example, a smart phone. In certain exemplary embodiments, this method is provided in the form of a video game, which makes the test more enjoyable to the subject undergoing the test. The method may include presenting a fixation target at a known location on a virtual reality display screen, wherein the fixation target is presented to both eyes stereoscopically; determining if subject's head has rotated to align a central site on the virtual reality display screen with the fixation target within a predetermined variable time window target; providing a visual stimulation target at a known location on the virtual reality display screen, wherein the visual stimulation target is presented to a single eye of the subject for a predetermined fixed amount of time Stimulus; determining a positive stimulation target detection by detecting head rotation of the subject in response to providing the visual stimulation target at the known location; storing the known location of the positive visual stimulation target detection; and repeating the above steps to construct a visual field map of a threshold of perception for the subject from the known locations of the positive visual stimulation target detection, thereby determining a subject's visual field perimetry with virtual reality.

US2014327880-A1 discloses a portable VOG device that facilitates the effective and efficient screening for TBI in military personnel in forward deployed military settings or remote locations using minimally trained staff. This includes the establishment of a protocol including optokinetic testing that will provide cost effective pre-screening of military personnel prior to deployment to establish a baseline of brain function prior to possible future injury. The efficiency of the device promotes subsequent follow-up screening to assess the effectiveness of prescribed TBI treatment.

### Description of the Invention

An object of the present invention is the provision of a portable instrument for diagnosis and rehabilitation of the visual field, aimed at patients with vision problems as a result of stroke, among other neural disorders.

Embodiments of the present invention provide, according to one aspect, a visual field rehabilitation device, comprising, as known in the field, a visual field capture component and an indicator device such a mouse or a joystick, among others.

The visual field capture component include a case, particularly made of polyamide, polypropylene or combinations thereof, among other types of materials, which comprises a first opening adaptable to the face of a user and a second opening adaptable for the arrangement and support of a portable computer device, particularly a smart phone. The portable computer device provides a screen configured to show visual field rehabilitation exercises under the command of a software application. The visual field capture component also includes a fastening element to allow the case to be tied up to the face of the user.

The indicator device is operatively connected to the software application and is configured to receive an input from the user when the user identifies a light stimulation, particularly a spot, on the screen.

Unlike the known proposals, the visual field capture component also includes one lens and a support for said lens. The support is configured to locate the lens facing the eyes of the user via movement thereof in order the exercises being carried out independently for each eye. The lens used by the present invention is a plane-convex lens with a spherical geometry. Therefore, the lens allows a complete field of view of the user and a complete view of the screen while the user is carrying out the visual field rehabilitation exercises.

In addition, the software application is configured to capture said input performed by the user and to automatically (i.e. in real-time) modify the visual field rehabilitation exercises, independently for each eye, based on a result of said capture. That is, the software application continuously adapts the visual field rehabilitation exercises depending on the result of the capture, i.e. of the campimetry measure performed by the user.

Moreover, the proposed device operates exclusively in the impaired visual area being able to detect the existence or absence of hemianopsias and quadrantanopsias.

The lens may have different diopters. In an embodiment the lens has diopters comprised in the range of 6 and 18 diopters, preferably between 8 and 15 diopters, and more preferably between 10 and 13 diopters. The lens may also have different diameters. In an embodiment the lens has a diameter in a range between 10 and 60 mm, preferably between 20 and 50 mm and more preferably between 30 and 40 mm. In a particular embodiment, the lens is of 12 diopters and has a diameter of 35 mm.

In an embodiment, the lens also has a selective absorption filter with a cutting frequency in a range between 100 and 600 nm, preferably between 200 and 500 nm, and more preferably between 350 and 450 nm. In a particular embodiment the lens comprises a selective absorption filter with a cutting frequency at 400 nm.

In a particular embodiment, the lens is arranged at a distance in a range between 6 and 10,5 cm from the screen, preferably between 7,5 and 9,5 cm.

In an embodiment, the lens is associated to a focusing gear mechanism configured to focus the lens. The assembly of the lens and focusing gear mechanism is configured to move with said support. The focusing gear mechanism in a particular embodiment is embodied as a gearwheel mechanism.

Present invention also proposes, according to another aspect, a method for visual field rehabilitation, the method comprises using the visual field rehabilitation device of the first aspect of the invention and:
- measuring the field of vision of a user's first eye by capturing, by the software application executed on the portable computer device, an input performed by the user via the indicator device, said input being made upon the user has identified a light stimulation such as a spot on the screen of the portable computer device using the first eye;
- measuring the field of vision of a user's second eye by capturing, by the software application, an input performed by the user via the indicator device, said input being made upon the user has identified a light stimulation on the screen of the portable computer device via the second eye; and
- automatically (i.e. in real-time) modifying a visual field rehabilitation exercise, independently for each eye, based on a result of said measures.

Other embodiments of the invention that are disclosed herein also include software programs to perform the method embodiment steps and operations summarized above and disclosed in detail below. More particularly, a computer program product is one embodiment that has a computer-readable medium including computer program instructions encoded thereon that when executed on at least one processor in a computer system causes the processor to perform the operations indicated herein as embodiments of the invention.

Present invention allows the user to carry out the visual field rehabilitation exercises without the help of a doctor, for example while the user is at its home. Moreover, the invention provides personalized visual field rehabilitation exercises taking at all times a current field of view of each eye, independently.

### Brief Description of the Drawings

The previous and other advantages and features will be more fully understood from the following detailed description of embodiments, with reference to the attached figures, which must be considered in an illustrative and non-limiting manner, in which:
Fig. 1 illustrates an embodiment of the proposed visual field rehabilitation device.
Figs. 2A and 2B schematically illustrates different views of the visual field capture component included in the proposed visual field rehabilitation device, according to an embodiment of the present invention.

### Detailed Description of a Preferred Embodiment

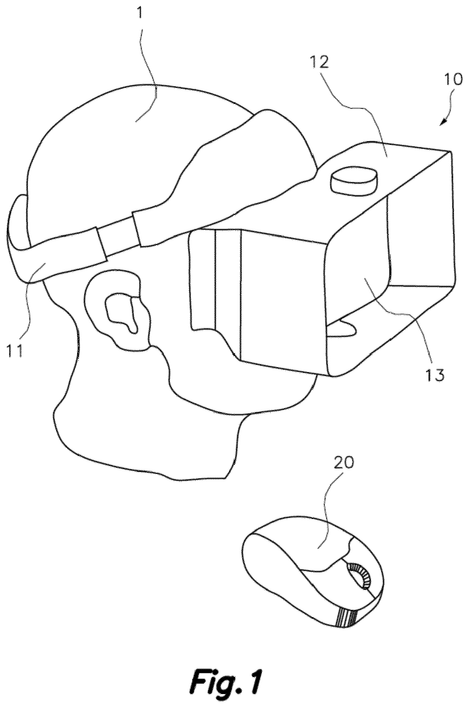

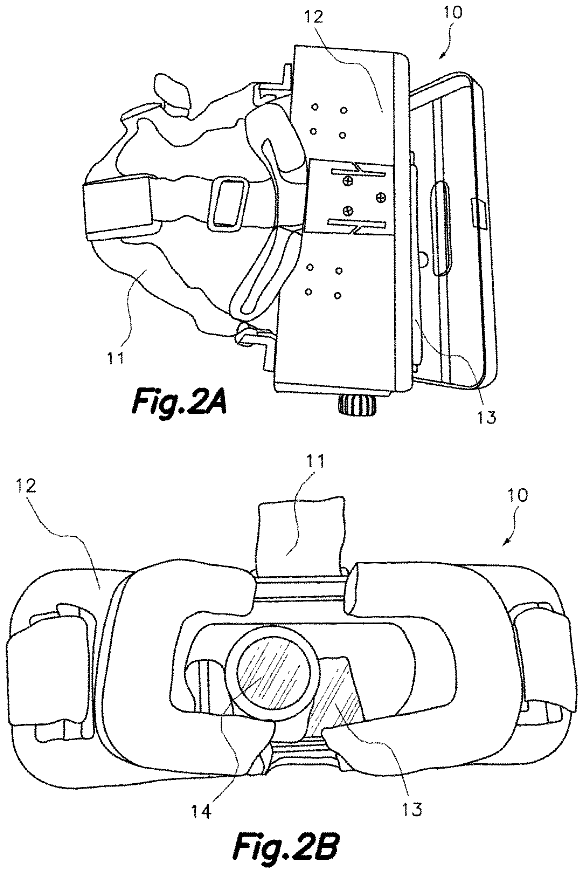

Figs.1 and 2 show a preferred embodiment of the proposed device 1. According to this embodiment, the device, which can also be referred as a kit, comprises a visual field capture component 1, in the form of a head-mounted member, and an indicator device 20 such as a mouse or joystick. The case 12 has an opening for the insertion of a portable computer device 13, in particular a 5.5 inches smart phone (not limitative as other sizes or types of portable computer devices could be used). The case 12 is specifically designed to be used by all kind of nasal anatomies.

The case 12 also holds one lens 14 arranged on a support (not illustrated). The support is movable inside the case 12 allowing the location of the lens 14 from one eye to the other. A fastening element 11, in this case embodied as a retaining strap, attached to the case 12, allows the latter to be tied up to the face of the user 1.

The portable computer device 13 has installed therein a software application executing visual field rehabilitation exercises. The portable computer device 13 is detachably attached to the visual field capture component 1 through a rear support (not illustrated), made of a metal or a plastic material. The rear support also allows the movement of the portable computer device 13 from one side to the other when changing the eye.

The lens 14 is a plane-convex spherical geometry lens. The plane side of the lens 14 is the closest to the eye when the user 1 is carrying out the visual field rehabilitation exercises.

In this particular embodiment, the lens 14 is a +12.00 diopter organic lens with a diameter of 35mm (it should be noted that other diopters and diameters can be used, for example the lens in other embodiments may have diopters in a range between 8 and 15 and diameters between 10 and 60 mm). The lens 14 particularly also has a selective absorption filter with a cutting frequency comprised in the range of 200 and 500 nm, more particularly at 400nm.

An optimum distance between the lens 14 and the screen of the portable computer device 13 is the one that makes it coincide with a focus object of the lens 14, which in this particular case is 8.33 cm from an anterior vertex of the lens 14. To allow the user 1 to autofocus, the distance may vary between 6 cm and 10.5 cm, more preferably between 7.5cm and 9.5cm. This movement varies the visual fields examined between 49.3º and 55.8º for the horizontal field and between 33.2º and 39.7º for the vertical field.

The support also has a focusing gear mechanism embodied as a gearwheel mechanism allowing the focus of the lens 14 by the user 1. The change from one eye to the other involves the movement of the support that holds the lens 14 and so the movement of the focusing gear mechanism.

In order for the portable computer device 13 to be fully visible through the lens 14 and for the user 1 to be able to focus correctly with the gearwheel mechanism, the eye must be as close as possible to the lens 14. Changing the lens support allows the lens 14 to be closer to the eye in a more natural way, so the user 1 can see the full screen of the portable computer device 13. In addition, for the eye not facing the lens 14, the change from color to black makes the contrast on the screen higher and the blind eye can be open in most cases.

A method for visual field rehabilitation is also proposed. In an embodiment, the method comprises measuring the field of vision of the user 1 via a campimetry. To do so, firstly a measure of the field of vision of the right eye of the user 1 is made by the software application capturing an input performed by the user 1 via the indicator device 20, said input being made upon the user 1 has identified a light stimulation, for example a spot, on the screen of the portable computer device 13. The software application automatically modifies a visual field rehabilitation exercise to be carried out by the user 1 using the right eye only based on the previous measure. After the user 1 has finished the visual field rehabilitation exercises for the right eye the lens 14 is moved/changed to the left eye of the user 1. Following, a measure of the field of vision of the left eye is made by the software application capturing an input performed by the user via the indicator device 20, said input being made upon the user 1 has identified a light stimulation, for example a spot, on the screen of the portable computer device 13. The software application automatically modifies a visual field rehabilitation exercise to be carried out by the user 1 using the left eye only based on the previous measure. Hence, with the proposed method the visual field rehabilitation exercises to be carried out by the user 1 for each of the eyes are shown via the screen of the portable computer device 13 taking at all times the current field of view of each eye.

The proposed method and corresponding device ensures the correctness of the measures that have been made by also controlling the detection of light stimuli in the blind spot. If the user 1 responds to stimuli in the blind spot, it means that the user 1 has looked away and therefore the measure is not valid. Hence the measure is discarded.

It should be noted that before each of the measures is performed, the user 1 may have to correctly focus the lens 14 and adjust, if required, the distance between the lens 14 (and so the support) and the portable computer device 13.

In an embodiment, the parameters involved in said campimetry are:
- Stimulus size: 1mm², equivalent to Goldmann perimeter size II.
- Duration of the stimulus: Between 0.2 and 0.1 seconds, being inferior to the latency time of the ocular movements that is of 0.25 seconds, so that the user does not have time to look away.
- Blind Spot Identification: Will be approximately 26.8mm to the right of the fixing point when the screen is 8.5cm from the eye, 23.6mm when it is 7.5cm and 29.9mm when it is 9.5cm.

The proposed method takes into account that the non-vision of the user 1 is not linear, but is an area of the visual field and that the user 1 is improving in his/her rehabilitation, so that the sessions, even if the exercises are the same, start from a more advanced point to be more efficient. The proposed method may further include follow-up exercises and scanning that makes the user 1 aware of his/her campimetric deficit so that he/she can use scanning strategies in his/her daily life. In addition, the proposed method may also include restoration exercises to recover the affected campimetric area. The recovery comes from the central area towards the periphery, so the exercises present stimuli in the affected campimetric limits.

Various aspects of the method and/or other processes, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at any time for the software programming.

A machine-readable medium may take many forms, including but not limited to, a tangible storage medium, a carrier wave medium or physical transmission medium. Non-volatile storage media include, for example, optical or magnetic disks, such as any of the storage devices in any computer(s), or the like, which may be used to implement the system or any of its components shown in the drawings. Volatile storage media may include dynamic memory, such as a main memory of such a computer platform. Tangible transmission media may include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media may include, for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a physical processor for execution.

The present disclosure and/or some other examples have been described in the above. According to descriptions above, various alterations may be achieved. The topic of the present disclosure may be achieved in various forms and embodiments, and the present disclosure may be further used in a variety of application programs. All applications, modifications and alterations required to be protected in the claims may be within the protection scope of the present disclosure.

The scope of the present invention is defined in the following set of claims.

## Claims

1. A visual field rehabilitation device, comprising:
- a visual field capture component (10) including:
- a case (12) comprising a first opening adaptable to the face of a user(1) and a second opening adaptable for the arrangement and support of a portable computer device (13), the portable computer device (13) providing a screen configured to show visual field rehabilitation exercises under the command of a software application; and
- a fastening element (11) configured to allow the case (12) to be tied up to the face of the user (1); and
- an indicator device(20) operatively connected to the software application and configured to receive an input from the user (1) when the user (1) identifies a light stimulation on the screen;
**characterized in that:**
- the visual field capture component (10) further includes one lens (14) and a support for said lens (14),
wherein the support is configured to locate the lens (14) facing one or the other eye of the user (1) via movement of the support in order the exercises being carried out independently for each eye;
wherein the lens (14) comprises a plane-convex lens with an spherical geometry, whereby the lens (14) provides a complete field of view of the user (1) and a complete view of the screen while the user (1) is carrying out the visual field rehabilitation exercises, and
- the software application is configured to capture said input performed by the user (1) and to automatically modify the visual rehabilitation exercises, independently for each eye, based on a result of said capture.

2. The device of claim 1, wherein said lens (14) is of a range between 6 and 18 diopters, preferably between 8 and 15 and more preferably between 10 and 13 diopters, and has a diameter in a range between 10 and 60 mm, preferably 20 and 50 mm and more preferably between 30 and 40 mm.

3. The device of claim 1 or 2, wherein the lens (14) comprises a selective absorption filter with a cutting frequency in a range between 100 and 600 nm, preferably between 200 and 500 nm and more preferably between 350 and 450 nm.

4. The device of any of previous claims, wherein the lens (14) is arranged at a distance in a range between 6 and 10,5 cm, preferably between 7,5 and 9,5 cm, from the screen.

5. The device of claim 1, wherein the lens (14) comprises 12 diopters, a diameter of 35 mm, a selective absorption filter with a cutting frequency of 400 nm and is arranged at a distance of 8.3 cm from the screen.

6. The device of any of previous claims, wherein the lens (14) is associated to a focusing gear mechanism configured to focus the lens (14), wherein the assembly of the lens (14) and focusing gear mechanism is movable along said support.

7. The device of claim 6, wherein the focusing gear mechanism comprises a gearwheel mechanism.

8. The device of any of previous claims, wherein the case is made of polyamide, polypropylene or combinations thereof.

9. A method for visual field rehabilitation, the method comprising using the visual field rehabilitation device of any of claims 1 to 8 and:
- measuring the field of vision of a user's first eye by capturing, by the software application executed on the portable computer device (13), an input performed by the user (1) via the indicator device (20), said input being made upon the user (1) has identified a light stimulation on the screen of the portable computer device (13) using the first eye;
- measuring the field of vision of a user's second eye by capturing, by the software application, an input performed by the user (1) via the indicator device (20), said input being made upon the user (1) has identified a light stimulation on the screen of the portable computer device (13) via the second eye; and
- automatically modifying a visual field rehabilitation exercise, independently for each eye, based on a result of said measures.

10. A non-transitory computer readable medium comprising code instructions that when executed by a computer system implement the method of claim 9.
